# EUROPEAN PATENT APPLICATION

(11) **EP 4 052 750 A1**
(43) Date of publication of application: **07.09.2022**
(21) Application number: 21160724.7
(22) Date of filing: 04.03.2021
(51) Int. Cl.: A61M 16/04, A61B 1/00, A61B 1/04, A61B 1/267

(54) **ENDOTRACHEAL DEVICES AND METHODS FOR USING AN ENDOTRACHEAL DEVICE**

(71) Applicant: Musuku, Sridhar, Watervliet NY 12189 (US); Cherukupalli, Divya, Watervliet NY 12189 (US)
(72) Inventor: Musuku, Sridhar, Watervliet NY 12189 (US); Cherukupalli, Divya, Watervliet NY 12189 (US)
(74) Representative: Cabinet Laurent & Charras

(57) **Abstract**

Endotracheal devices and methods of using endotracheal devices are provided. The devices include a first elongated conduit having an internal dimension, a second elongated conduit having an external dimension less than the internal dimension of the first conduit, and a manipulation rod operatively mounted to the second conduit, where the manipulation rod allows an operator to move the second conduit within the first conduit. The manipulation rod may be used translate and/or rotate the second conduit within the first conduit. The first and second elongated conduits may be provided with inflatable cuffs to minimize the passage of fluids. Though aspects of the invention may be uniquely adapted for insertion through the trachea and into a bronchus, aspects of the invention maybe used for other bodily passages.

## Description

### BACKGROUND OF THE INVENTION

### Technical Field

This invention is generally related to endotracheal or intubation devices and the use of endotracheal or intubation devices, for example, in operating rooms, emergency rooms, and by first responders. Specifically, aspects of the invention include endotracheal devices having an external conduit, an internal conduit within the external conduit, and a rod that can be used to move the internal conduit within the external conduit to treat patients.

### Description of Related Art

Endotracheal tubes (or ETTs) are conventional supra-glottic (that is, above the glottic or laryngeal opening) devices used, for example, during surgery, to - among other things - maintain access to the trachea of the patient. Endotracheal tubes are also conventional tubes specially designed with inflatable cuffs to seal airways and effectively support oxygenation and ventilation, for example, during surgery.

In the existing art, especially during thoracic surgery, endotracheal tubes may be used to isolate one lung from the other. For example, a thoracic surgeon may require that the lungs be isolated and a single lung be ventilated when performing certain procedures, for instance, lobectomy, bronchopulmonary lavage, and esophagogastrectomy, among other procedures.

One device that is may be used to isolate the lungs is a relatively rigid tubular device referred to as "double-lumen endotracheal tube" (DLT). When using a DLT, the user, for example, an anesthesiologist, inserts a tube having two lumens into the trachea through which to ventilate the lungs. One of the two lumens is positioned in the left or right bronchial main stem and the other lumen remains in the trachea. However, though DLTs can provide an effective means for isolating a lung, DLTs can be bulky and thus cumbersome to manipulate by the user, such as, an anesthesiologist. In addition, DLTs typically must be left in place in the patient for a few days after surgery if the patient is transferred to intensive care after surgery.

However, it is well recognized in the art that in the use of ETTs the manipulation and positioning of the tube in the airway, for example, in a bronchus, can be difficult without undo diligence and care. Of course, these prior art devices and practices are being performed on patients, where misstep or error can be harmful, if not life threatening.

Aspects of the invention address these and other disadvantages of the prior art by providing a unique ETT that not only avoids the inconveniences and complications of the prior art, but also can provide unique opportunities for improved techniques and improved patient treatment and outcome.

### SUMMARY OF THE INVENTION

Aspects of the present invention may, for example, be used for lung isolation, among other procedures, and may facilitate the positioning and/or orienting of tubes within the trachea and bronchi. In addition, according to aspects of the invention, once the, for example, thoracic surgery is completed, the inner conduit or tube of the present invention can be withdrawn from the patient. In other aspects, the inner conduit or tube of the present invention may be left in place within the patient. Aspects of the present invention may also be less bulky and less cumbersome to handle and position within the patient compared to existing devices. In short, aspects of the present invention may be easier to use by the anesthesiologist and thus easier to isolate a lung of a patent than existing devices.

In the following discussion, aspects of the invention may be referred to as "endotracheal devices"; however, aspects of the invention may be referred to as "intubation devices" or "endobronchial devices," for example, depending upon the use, function, and/or positioning of the aspect disclosed.

Aspects of the present invention address the disadvantages of the existing art by providing endotracheal devices having two cooperating conduits or tubes, an external conduit and an internal conduit positioned within the external conduit, and a manipulation rod or wire mounted to the internal conduit which may be used to move the internal conduit, for example, telescopically, within the external conduit to treat patients. Embodiments of the invention and the many aspects the embodiments are summarized in the following descriptions.

One embodiment of the invention is an endotracheal device comprising or including: a first elongated conduit having an internal dimension, a proximal end, and an open distal end; a second elongated conduit having an external dimension less than the internal dimension of the first conduit, an open proximal end in fluid communication with the first elongated conduit, and an open distal end; and a manipulation rod operatively mounted to the second conduit, the manipulation rod adapted to allow an operator to move the second conduit within the first conduit. In one aspect, the manipulation rod may be adapted to allow the operator to translate the second conduit within the first conduit and/or rotate the second conduit within the first conduit.

In one aspect, the first conduit further comprises an opening through which the manipulation rod accesses the second conduit, wherein movement of the manipulation rod moves the second conduit within the first conduit. In one aspect, the opening may be an elongated slot, in another aspect, the opening may include a fluid-sealing element.

In anther aspect, the device may further include a rod receiver mounted to the first conduit, and the rod receiver may have an opening to receive the manipulation rod. The opening in the rod receiver may include a fluid sealing element. The opening in the rod receiver may a circumferentially directed slot.

In one aspect, the device may further include a projection, tab, or bar connecting the manipulation rod to the second conduit.

In another aspect, the device may further include at least one inflatable cuff mounted to one of the first conduit and the second conduit.

Another embodiment of the invention is a method for treating a patient. The method may comprise or include: introducing to a cavity within the patient a device comprising: a first elongated conduit having an internal dimension, a proximal end, and an open distal end; a second elongated conduit having an external dimension less than the internal dimension of the first conduit, an open proximal end in fluid communication with the first elongated conduit, and an open distal end; and a manipulation rod operatively mounted to the second conduit; and moving the second conduit within the first conduit with the manipulation rod to position the distal end of the second conduit within the patient. The step of moving the second conduit within the first conduit may comprise translating and/or rotating the second conduit within the first conduit.

In one aspect, the cavity within the patient being treated may be an airway, such as, a trachea or bronchus.

In another aspect, the method may further include introducing and/or extracting a fluid from the cavity.

In another aspect, the method may further include inflating at least one inflatable cuff on the first conduit and/or the second conduit to obstruct fluid flow passed the cuff.

These and other aspects, features, and advantages of this invention will become apparent from the following detailed description of the various aspects of the invention taken in conjunction with the accompanying drawings

### BRIEF DESCRIPTION OF THE DRAWINGS

The subject matter, which is regarded as the invention, is particularly pointed out and distinctly claimed in the claims at the conclusion of the specification. The foregoing and other objects, features, and advantages of the invention will be readily understood from the following detailed description of aspects of the invention taken in conjunction with the accompanying drawings in which:
FIGURE 1 is a side view of an endotracheal device according to one aspect of the invention.
FIGURE 2 is a top view of the endotracheal device shown in FIGURE 1.
FIGURE 3 is a detailed cross-sectional view of the endotracheal device shown in FIGURE 1 as identified by Detail 3 in FIGURE 1.
FIGURE 4 is a cross-sectional view of the endotracheal device shown in FIGURES 1 and 2 having the internal translatable conduit in a first position according to an aspect of the invention.
FIGURE 5 is a cross sectional view of the endotracheal device shown in FIGURE 4 having the internal translatable conduit in a second position according to an aspect of the invention.
FIGURE 6 is a partial view of the endotracheal device shown in FIGURES 1 through 5 positioned in an airway, shown in cross section, according to an aspect of the invention.
FIGURE 7 is a cross-sectional view of another endotracheal device according to an aspect of the invention.
FIGURE 8 is a side view of another endotracheal device according to an aspect of the invention.
FIGURE 9 is a top view of the endotracheal device shown in FIGURE 8.

### DETAILED DESCRIPTION OF ASPECTS OF THE INVENTION

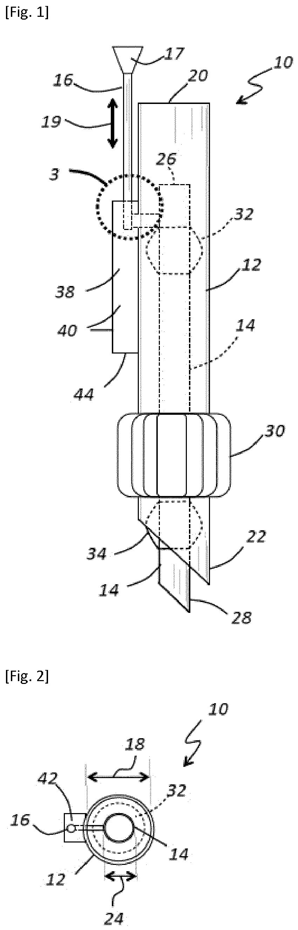

FIGURE 1 is a side view of an endotracheal device 10 according to one aspect of the invention. FIGURE 2 is a top view of the endotracheal device 10 shown in FIGURE 1. As shown, endotracheal device 10 typically incudes a first elongated conduit 12, second elongated conduit 14 positioned for movement within first elongated conduit, and a manipulation rod or wire 16 operatively mounted to the second conduit 14, the manipulation rod 16 is adapted to allow an operator to move the second conduit 14 within the first conduit 12. According to aspects of the invention, manipulation wire or rod 16 may be adapted to translate and/or rotate second conduit 14 within the first conduit 12.

In one aspect, an endotracheal device 10 may be referred to as "an endotracheal tube (ETT)" or "an endotracheal tube device." In another aspect, each of first elongated conduit 12 and second elongated conduit 14 may be referred to as "an endotracheal tube." For example, in one aspect, device 10 may be referred to as "an endotracheal tube within an endotracheal tube" or "an ETT within an ETT." It is envisioned that the inventors and/or others may conceive of other appropriate designations for aspects of the invention.

In one aspect, the first elongated conduit 12 typically has an internal dimension 18, for example, an inside diameter or inside width; a proximal end 20; and an open distal end 22; and second elongated conduit 14 typically has an external dimension 24, for example, an outside diameter or outside width, less than the internal dimension 18 of the first conduit 12; an open proximal end 26; and an open distal end 28. Typically, open proximal end 26 of second elongated conduit 14 may be in fluid communication with the interior of the first elongated conduit 12.

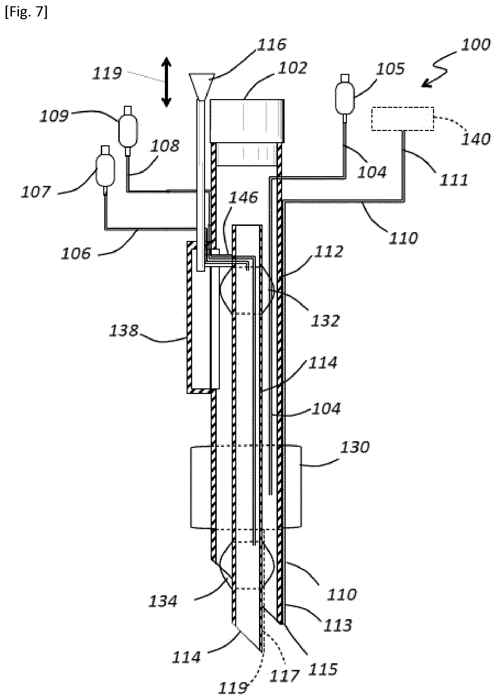

As shown in FIGURES 1 and 2, endotracheal device 10 may include one or more inflatable cuffs or balloon cuffs adapted to substantially obstruct the annular cavity in which the cuffs are located when inflated to prevent fluid flow past the balloon cuff. In one aspect, the first elongated conduit 12 may include one or more balloon cuffs 30, for example, at least one balloon cuff 30 located proximate the distal end 22 of conduit 12. According to aspects of the invention, cuff 30 (and any inflatable cuff disclosed herein) may operate and function as a conventional cuff as known in the art, for example, cuff 30 may inflate or expand when filled with a fluid, for example, pressurized gas, such as, air, and deflate when the fluid is depressurized or removed from the cuff 30. When inflated, balloon cuff 30 may obstruct the annular cavity between the outside surface of conduit 12 and the internal surface (not shown) of the cavity or passage into which conduit 12 is positioned. In one aspect, the second elongated conduit 14 may include one or more balloon cuffs 32, 34, for example, at least one balloon cuff 32 located proximate the proximal end 26 of conduit 14 and one balloon cuff 34 located proximate the distal end 28 of conduit 14. When inflated, balloon cuffs 32 and 34 may obstruct the annular cavity between the outside surface of conduit 14 and the internal surface of conduit 12 and/or the internal surface (not shown) of the cavity or passage into which conduit 14 is positioned. Balloon cuffs 30, 32, and 34 may be inflated via one or more conduits or lumens (not shown in FIGURES 1 and 2), as typical in the art. See FIGURE 7 for typical balloon cuff inflation conduits or lumens that may be used.

As shown in FIGURES 1 and 2, according to aspects of the invention, manipulation wire or rod 16 is typically connected to second conduit 14 and accesses conduit 14 via a rod receiver or rod channel 38. According to aspects of the invention, wire or rod 16 may be any elongated structure operatively connected to second conduit 14 whereby movement of rod 16 effects movement, for example, translation (as indicated by double arrow 19) and/or rotation of conduit 14. As shown in FIGURES 1 and 2, rod 16 may comprise an elongated cylindrical structure or element, for example, a circular cylindrical element, elliptical cylinder element, polygonal cylindrical element, and the like. Rod 16 may be solid or hollow, for example, tubular. Rod 16 may be relatively rigid or flexible. In one aspect, rod 16 may comprise a relatively rigid filament, a wire, or a thin rod. As shown in FIGURE 1, in one aspect, rod 16 may include a structure 17 adapted to facilitate handling and/or manipulation of rod 16, such as, a knob or a bulb. The rod 16 or the knob or bulb 17 may be knurled to facilitate handling.

Rod receiver 38 may typically comprise a structure or housing positioned and adapted to receive rod 16 and allow rod 16 to access and move conduit 14 within conduit 12. In one aspect, rod receiver 38 may be adapted to receive rod 16 and allow rod 16 to access and move conduit 14 while minimizing or preventing the passage of fluid from device 10, for example, from rod receiver 38 and/or conduit 12. As shown in FIGURES 1 and 2, rod receiver 38 may comprise a housing having sides 40, a top 42, and a bottom 44. Though, in one aspect, the housing of rod receiver 38 may be hexagonal or square in cross section, as shown in FIGURES 1 and 2, in another aspect, the housing of rod receiver 38 may be curved, for example, circular or elliptical in cross section.

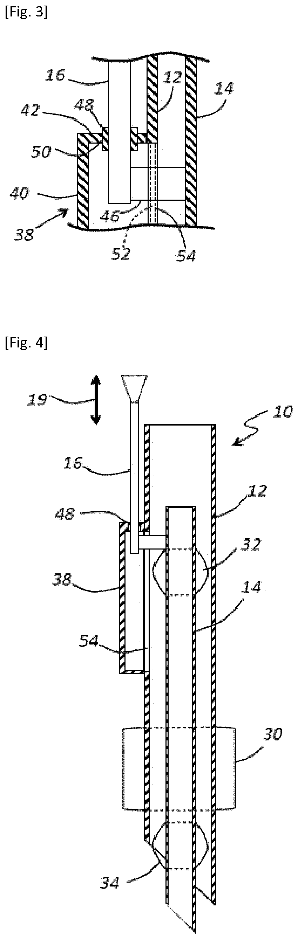

FIGURE 3 is a cross-sectional view of the Detail 3 shown in FIGURE 1, and illustrates one mechanism for mounting rod or wire 16 to second conduit 14 via rod receiver 38. According to aspects of the invention, any conventional means may be used for attaching rod 16 to second conduit 14 where the movement of rod 16 effects the movement of second conduit 14. As shown in FIGURE 3, in one aspect, rod 16 may be mounted to conduit 14 by one or more projections, tabs, plates, rods, or bars 46. The one or more projections 46 may be mounted to rod 16 and second conduit 14 by conventional means, for example, via mechanical fasteners or/ an adhesive. In one aspect, the one or more projections 46 may be integrally molded to rod 16 and/or to conduit 14.

As also shown in FIGURE 3, in one aspect, rod receiver 38 may include one or fluid sealing devices or elements 48, for example, one or more fluid sealing devices or elements 48 about a hole or slot 50 in top 42 of rod receiver 38 through which rod 16 passes into rod receiver 38. Device 10 may also include one or more sealing devices 52 (shown in phantom in FIGURE 3) about an aperture or slot 54 between rod receiver 38 and conduit 12. According to aspects of the invention, the aperture of slot 54 may provide a passage for rod 16 to access conduit 14.

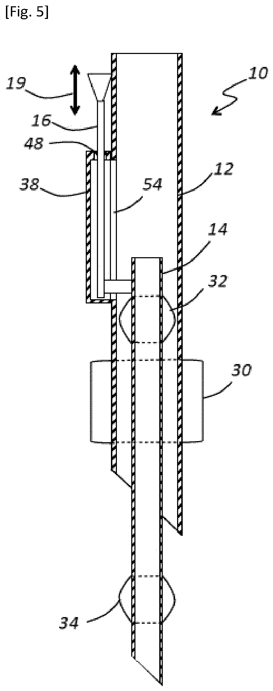

FIGURE 4 is a cross sectional view of the endotracheal device 10 shown in FIGURES 1, 2, and 3 having the internal translatable conduit 14 in a first position according to an aspect of the invention. FIGURE 5 is a cross sectional view of the endotracheal device 10 shown in FIGURE 4 having the internal translatable conduit 14 shown in a second position, different from the first position shown in FIGURE 4, according to an aspect of the invention.

As shown in FIGURES 4 and 5, according to aspects of the invention, an operator, for example, an anesthesiologist (though aspects of the invention may be used by, for example, any health care professional or first responder), may manipulate rod 16 as indicated by double arrow 19, to translate rod 16 and internal second conduit 14 from, for example, the first position shown in FIGURE 4, to the second position shown in FIGURE 5. For example, in one aspect of the invention, an operator may first position device 10 in an airway, for example, through the trachea of a patient, and while the second conduit 14 is positioned in the first position shown in FIGURE 14, then, with or without the inflation of cuffs 30, 32, and/34, advance the position of internal second conduit 14 from the first position shown in FIGURE 4 to the second position shown in FIGURE 5 using the rod 16. The translation or advancement of rod 16 and conduit 14 may be practiced with or without visual videographic or x-ray imaging assistance, and/or with or without the use of a guiding device, such as, a "bougie," as known in the art.

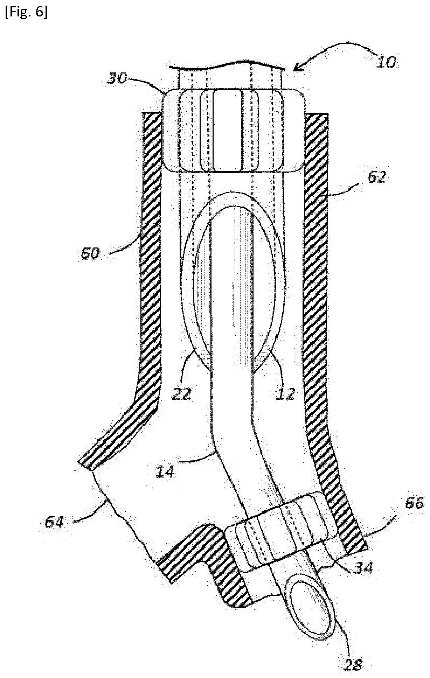

FIGURE 6 is a partial view of the endotracheal device 10 shown in FIGURES 1 through 6 positioned in an airway 60, shown in cross section, according to an aspect of the invention. In this non-limiting depiction of one use of an aspect of the invention, the airway 60 shown in FIGURE 6 includes a portion of a trachea 62, for example, a human trachea (also known as "the windpipe"), and portions of the right main bronchus 64 and left main bronchus 66 leading to the lungs (not shown).

As shown in FIGURE 6, according to one aspect of the invention, with the use of manipulation rod 16 (not shown in FIGURE 6), the inner conduit 14 of endotracheal device 10 may be extended from, for example, the outer conduit 12, and directed into one of the bronchi 64 or 66. As shown in FIGURE 6, inflation cuff 30 about conduit 12 may be inflated against the inner surface of trachea 62 to minimize or prevent passage of fluids, such as, bodily liquids or gases, through the annular cavity about conduit 12. In addition, inflation cuff 34 about conduit 14 may be inflated against the inner surface of left main bronchus 66 to minimize or prevent passage of fluids, such as, bodily fluids or gases, between the annular cavities about conduit 14.

Though one specific airway that may be accessed by aspects of the invention is shown in FIGURE 6, according to other aspects of the invention, endotracheal device 10 may be used in any human or animal cavity, passage, or airway to access and isolate portions of the cavity, passage, or airway. For example, in one aspect of the invention, device 10 may be used to isolate a lung during thoracic surgery, for example, during a lobectomy, a pneumonectomy, a pleural decortication, a bullectomy, bronchopulmonary lavage, an esophagogastrectomy, a thymectomy, or a mediastinal mass resections, among thoracic surgeries.

FIGURE 7 is a cross-sectional view of another endotracheal device 100 according to an aspect of the invention. Endotracheal device 100 may include any one or more of the aspects and features of endotracheal device 10 shown in FIGURES 1 through 6. Among other things, endotracheal device 100 discloses several ancillary features that may be provided for aspects of the invention, including balloon cuff inflation conduits and fluid sampling or venting conduits.

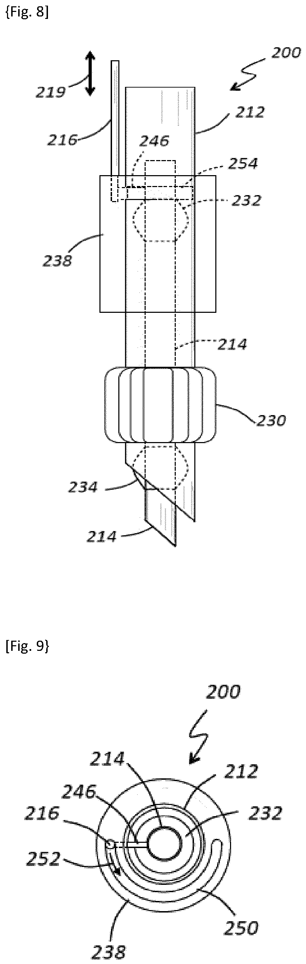

As shown in FIGURE 7, in one aspect, device 100 may include the features disclosed for device 10, including a first external conduit 112 and second internal conduit 114, and one or more manipulation rods or wires 116 mounted to internal conduit 114 and translatable, as indicated by double arrow 119, and/or rotatable (see FIGURES 8 and 9), and a rod receiver 138, as disclosed herein. Rod 116 may include a mounting bar or tab 146 that connects rod 116 to conduit 114. According to the aspect shown in FIGURE 7, device 100 may also include one or more balloon cuffs 130 about conduit 112, and one or more balloon cuffs 132, 134 about conduit 114, as disclosed herein.

As shown in FIGURE 7, device 100 may include one or more connectors or couplings 102 adapted to engage one or more conduits in fluid communication with an external fluid source (not shown) or an external fluid receiver (not shown). For example, in one aspect, coupling 102 may be in fluid communication with a source of treatment fluid, for example, an oxygen-containing gas. Coupling 102 may be a conventional 15 mm connector, and may be threadably mounted to the proximal end of conduit 112.

As shown in FIGURE 7, device 100 may include inflation conduits or lumens 104, 106, and 108 in fluid communication with the inflation cuffs 130, 132, and 134, respectively. As is conventional, lumens 104, 106, and 108 may be in fluid communion with a source of pressurized gas, such as, air, to inflate or deflate cuffs 130, 132, and 134, respectively. As shown in FIGURE 7, in one aspect, lumens 104, 106, and 108 may be operatively connected to inflation pilot balloons 105, 107, and 109, respectively, as known in the art, though any source of pressurized gas may be used to inflate inflation cuffs 130, 132, and 134.

As shown, inflation lumen 104 associated with cuff 130 may be mounted to or within and extend along conduit 112.

According to aspects of the invention shown in FIGURE 7, since cuffs 132 and 134 may be mounted and move, for example, translate and/or rotate, with conduit 114, inflation lumens 106 and 108 may be mounted to or within conduit 114 and be adapted to maintain fluid communication with cuffs 132 and 134 with the movement of conduit 114. In one aspect of the invention, lumens 106 and 108 may pass through conduit 112 and/or rod receiver 138 and then mount to and extend along conduit 114 to access cuffs 132 and 134, respectively, while allowing for the movement of conduit 114. In one aspect, a portion of lumen 106 and/or a portion of lumen 108 may be unmounted and be allowed to freely flex and/or deflect along an unsecured length of lumens 106 and/or 108 within device 100 such that lumens 106 and 108 do not restrict or interfere with the movement of conduit 114. In one aspect, lumen 106 and/or lumen 108 may access conduit 114 as disclosed in pending U.S. application 16/255,983, the disclosure of which is included by reference herein. For example, lumen 106 and/or lumen 108 may pass along or within rod 116, pass across or within projection 146, and then along or within conduit 114 to access cuff 132 and/or cuff 134.

According to one aspect of the invention, as shown in FIGURE 7, lumens 106 and 108 may access conduit 114 via tab or bar 146 mounted between rod 116 and conduit 114 where lumens 106 and 108 move with rod 116 and bar 146.

In one aspect of the invention, device 100 may include one or more other ancillary lumens or conduits 110 having a proximal end 111 and a distal end 113. For example, in one aspect, one or more lumens or conduits 110 may include one or more open ends, holes, or orifices 115 adjacent to distal end 113. According to aspects of the invention, the one or more orifices or holes 115 may be provided in or on conduit 112 (for example, evenly distributed in, about and/or along the distal end of conduit 112). In one aspect, one or more lumens or conduits 110 may communicate via proximal end 111 with one or more external fluid sources (not shown) and/or one or more external fluid receivers (not shown). According to aspects of the invention, the one or more lumens or conduits 110 may be used to introduce one or more fluids to or extract one or more fluids from an internal cavity or passage, such as, a trachea, a bronchus, an esophagus, or another cavity or passage into which device 110 is placed. The proximal ends 111 of one or more lumens or conduits 110 may communicate with external sources, for example, sources of medication, or treatments, for example, vacuum or fluid pressure, by conventional means. In one aspect, lumens or conduits 110 may be used to inflate or deflate an internal cavity, for example, a lung.

In one aspect of the invention, lumen or conduit 110 and hole 115 and/or conduit 117 and hole 119 may be associated with an image-capturing device 140, shown in phantom in FIGURE 9. For example, in one aspect, hole 15 and/or hole 119 may be image capturing apertures, conduit 110 and/or conduit 115 may be one or more wires or cables, for example, a fiber optic cable, adapted to transmit signals corresponding to the images captured by the aperture 115 and/or aperture 119 to image capturing device 140. The images captured may be still images or moving images. Image capturing device 140 may be any device adapted to receive and store, locally or remotely, signals associated with the images captured by aperture 115 and/or aperture 119. Image capturing aperture 140 may be adapted to capture images in the visual spectrum, or in any range of the electromagnetic spectrum desired, including x-ray images, infrared images, and microwave images, and the like.

In another aspect, one or more lumens or conduits 117 (only a representative portion of which is shown in phantom in FIGURE 7) may be associated with internal conduit 114 and have one or more orifices 119 in the distal end of internal conduit 114 to provide similar functions as lumens 110, for example, the introduction or removal of a fluid from an internal cavity adjacent the distal end of conduit 114. In one aspect, the one or more lumens or conduits 117 having one or more orifices 119 (shown in phantom) may be associated with internal conduit 114 and may be adapted to accommodate the movement of internal conduit 114 in a fashion similar to lumens 106 and 108, as disclosed herein. For example, the one or more lumens or conduits 117 associated with internal conduit 114 may include a portion that is unmounted and freely flexes along an unsecured length such as to not restrict or interfere with the movement of conduit 114, and/or the one or more lumens or conduits 117 associated with internal conduit 114 may access conduit 114 via tab or bar 146 mounted between rod 116 and conduit 114 and move with rod 116 and bar 146.

FIGURE 8 is a cross sectional view of another endotracheal device 200 according to an aspect of the invention. FIGURE 9 is a top view of the endotracheal device 200 shown in FIGURE 8.

As shown in FIGURES 8 and 9, in this aspect, device 200 (and device 10] and device 100) may include the features disclosed for device 10, including a first external conduit 212 and second internal conduit 214, and one or more manipulation rods or wires 216 mounted to internal conduit 214 and translatable (as indicated by double arrow 219), and a rod receiver 238, as disclosed herein. Rod 216 may include one or more mounting projections, bars, or tab 246 that connects rod 216 to conduit 214. According to the aspect shown in FIGURES 8 and 9, device 200 may also include one or more balloon cuffs 230 about conduit 212, and one or more balloon cuffs 232, 234 about conduit 214, and appropriate cuff inflation lumens (not shown), as disclosed herein.

As shown in FIGURES 8 and 9, in this aspect, device 200 is adapted to rotate and/or translate internal conduit 214 within external conduit 212 by means of rod 216. As shown most clearly in FIGURE 9, in this aspect, rod receiver 238 comprises a cylindrical housing that at least partially surrounds external conduit 212 and provides a channel or slot 250 for the movement of rod 216. In this aspect, the shape and location of slot 250 in rod receiver 238 allows an operator to translate rod 216 within slot 240, as indicated by arrow 252, and thereby rotate internal conduit 214 within external conduit 212. In a fashion similar to hole 50 shown in FIGURE 3, slot 250 may include a sealing device or sealing element (not shown) adapted to minimize or present leakage of fluid through slot 250. In the aspect shown in FIGURES 8 and 9, conduit 212 may include one or more circumferentially directed passages or slots 254 (shown in phantom) to allow passage of projection 246 about conduit 212 during the arcuate translation of rod 216 within slot 250.

In one aspect of the invention, in order to allow the translation and/or rotation of internal conduit 214 within external conduit 216 by means of rod 246, the external conduit 212 may be omitted within the extents of the housing of rod receiver 238 allowing rod 216 and, for example, projection 246 unencumbered access to internal conduit 214 to translate and/or rotate internal conduit 214. According to this aspect, the housing of rod receiver 238 may provide a chamber for accessing internal conduit 214 with rod 216 and allowing rod 216 and one or more projections 246 unencumbered access to conduit 214, while providing appropriate sealing devices or elements about any penetrations into the housing of rod receiver 238.

According to aspect of the invention, the arc length of slot 250 may range from 5 degrees to 360 degrees, for example, from 15 degrees to 270 degrees, or from 5 degrees to 180 degrees, as suggested by the aspect shown in FIGURE 9.

The use of any one of the endotracheal devices disclosed herein may be practiced with or without the use of a guidance device, that is, a device used to assist the user in guiding the insertion and placement of the endotracheal devices disclosed herein. According to aspects of the invention, any conventional guidance device may be used, including a thin surgical instrument, or "bougie," as known in the art, that is, a "hard bougie" or a "soft bougie"; a bronchoscope, for example, a fiber optic bronchoscope; or any elongated rod, tube, wire, conduit, or structure that can be used to assist the user in guiding the insertion and placement of the endotracheal devices disclosed herein. According to one aspect of the invention, the guidance device may be placed within the passage or airway of the patient, and, while in place, any one of the endotracheal devices disclosed herein may be inserted into the airway while threading the guidance device through, for example, the internal conduit of an endotracheal device disclosed herein. Guidance devices that may be used when practicing aspects of the invention may include an Eschmann-style tracheal tube introducer or bougie provided by various manufactures, such as, Teleflex Inc. Insertion of aspects of the invention into a passage or an airway may be facilitated by lubricating the endotracheal device prior to insertion.

Though many different aspects of the present invention have been presented disclosed individually or in combination herein for the sake of facilitating disclosure, it is envisioned that any one or more of the aspects or features disclosed herein may be combined with any one or more other aspects of features disclosed herein.

The endotracheal devices and their subcomponents disclosed herein may be fabricated from any conventional material, for example, any conventional plastic material, elastomeric material, and even wood or metal. In one aspect, the materials from which the components of the invention, for example, the first conduit, the second conduit, and/or the manipulation rod, are made may provide for at least some flexibility, deformability, and/or malleability to the components. That is, in some aspects, the components, such as, the first conduit, the second conduit, and/or the manipulation rod, may be bent, deformed, or otherwise manipulated during handling by an operator, such as, an anesthesiologist, to introduce, position, and/or orient the devices as desired within a cavity or passage of a patient, for example, in an airway. In one aspect, the components disclosed herein may be fabricated from a polyamide (PA), for example, nylon; a polyethylene (PE), both high-density polyethylene (HDPE) and low-density polyethylene (LDPE); a polyethylene terephthalate (PET); a polypropylene (PP); a polyester (PE); a polytetrafluoroethylene (PTFE); a polystyrene (PS); an acrylonitrile butadiene styrene (ABS); a polycarbonate (PC); or a polyvinylchloride (PVC); among other plastics.

The endotracheal devices disclosed herein may also be provided in a broad range of sizes and dimensions, for example, depending upon the size or age of the patient being treated. Aspects of the invention may be adapted for use with adults, children, infants, and neonates. In one aspect, embodiments of the invention may be used for veterinary treatment, for example, with animals. For example, the endotracheal devices may have an overall length ranging from about 2 inches to about 2 feet, but may typically range in length from about 4 inches to about 12 inches, for example, about 10 inches in length. Similarly, the endotracheal devices disclosed herein may have an overall width ranging from about 1 inch to about 6 inches, but may typically range in width from about 2 inches to about 3 inches, for example, about 2½ inches in width.

The internal conduits and the external conduits of the devices disclosed herein may have cross-sectional dimensions, for example, diameters or widths, ranging from 2 millimeters [mm] to 100 mm, but typically have cross-sectional dimensions ranging from 5 mm to 20 mm, for example, from 8 mm to 12 mm. For instance, in one aspect, the internal diameter of the external conduit (12) disclosed herein may be 9 mm, and the internal diameter of the internal conduit (14) disclosed herein may be 6 mm.

Accordingly, in one aspect of the invention, any one of the distal ends of the conduits or tubes disclosed herein may be substantially non-beveled, for example, having an exposed cross-section substantially perpendicular to the axis of the tube. In another aspect, any one of the distal ends of the tubes or conduits disclosed herein may have a bevel, directed in a predetermined direction, for example, having a bevel directed in an anterior direction of the patient. The bevel may have a bevel angle ranging from 30 degrees to 60 degrees, for example, a 45-degree bevel.

Though various aspects and embodiments of the invention are disclosed herein that can be used or adapted for endotracheal insertion or intubation during surgery, it is envisioned that aspects of the invention may be adapted for any application where patient or victim treatment or intubation is advantageous, that includes in the emergency room (ER), in the operating room (OR), or by first responders, paramedics, and emergency medical technicians (EMTs) at accidents or other calamities. It is envisioned that aspects of the invention may be particularly advantageous for neurosurgery and/or brain surgery where the prevention of coughing, which may undesirably induce intra-cranial pressure (ICP) during extubation, is desired. Other applications of aspects of the invention are bronchoscopy thoracic surgery, obstetrics, cardiac catheterization, laparoscopic procedures, and plastic surgery, among other medical procedures, and training, for example, training of anesthesiologists.

As disclosed herein, aspects of the invention include endotracheal devices and their method of use that address many of the disadvantages of prior art devices and methods.

While various embodiments have been described above, it should be understood that these embodiments and their many aspects have been presented by way of example, and not limitation. It will be apparent to persons skilled in the relevant art that various changes in form and detail can be made therein without departing from the spirit and scope of the invention. Moreover, it is to be understood that the various embodiments of the invention, although different, are not necessarily mutually exclusive. Furthermore, a particular feature, structure, or characteristic described herein in connection with one embodiment may be implemented within other embodiments without departing from the scope of the invention. In addition, it is to be understood that the location or arrangement of individual elements within each disclosed embodiment may be modified without departing from the scope of the invention. The detailed description presented herein, therefore, is not to be taken in a limiting sense, and the scope of the present invention is defined only by the appended claims, appropriately interpreted, along with the full range of equivalents to which the claims are entitled.

Although the term "at least one" may often be used in the specification, claims and drawings, the terms "a", "an", "the", "said", etc. also signify "at least one" or "the at least one" in the specification, claims and drawings.

While several aspects of the present invention have been described and depicted herein, alternative aspects may be effected by those skilled in the art to accomplish the same objectives. Accordingly, it is intended by the appended claims to cover all such alternative aspects as fall within the true spirit and scope of the invention.

## Claims

1. An endotracheal device (10) comprising:
a first elongated conduit (12) having an internal dimension, a proximal end, and an open distal end;
a second elongated conduit (14) having an external dimension less than the internal dimension of the first conduit, an open proximal end in fluid communication with the first elongated conduit, and an open distal end; and
a manipulation rod (16) operatively mounted to the second conduit (14), the manipulation rod adapted to allow an operator to move the second conduit (14) within the first conduit (12).

2. The device as recited in claim 1, wherein the manipulation rod (16) is adapted to allow the operator to one of translate the second conduit (14) within the first conduit (12) and rotate the second conduit (14) within the first conduit (12).

3. The device as recited in claim 1 or claim 2, wherein the first conduit (12) further comprises an opening through which the manipulation rod (16) accesses the second conduit (14), wherein movement of the manipulation rod moves the second conduit within the first conduit.

4. The device as recited in claim 3, wherein the opening comprises an elongated slot (54).

5. The device as recited in claim 3, wherein the opening comprises a fluid sealing element (48).

6. The device as recited in any one of claims 1 to 5, wherein the device further comprises a rod receiver (38) mounted to the first conduit (12), the rod receiver having an opening (50) to receive the manipulation rod (16).

7. The device as recited in claim 6, wherein the opening (50) to receive the manipulation device (16) comprises fluid sealing element (48).

8. The device as recited in claim 6, wherein the opening (50) in the rod receiver (38) comprises a circumferentially-directed slot (254).

9. The device as recited in any one of claims 1 to 8, wherein the device further comprises a projection (46) connecting the manipulation rod to the second conduit.

10. The device as recited in any one of claims 1 to 9, wherein the device further comprises an at least one inflatable cuff (30, 32, 34) mounted to one of the first conduit and the second conduit.

11. The device as recited in any one of claims 1 to 10, wherein the device further comprises an image capturing device (140).

12. The device as recited in any one of claims 1 to 11, wherein the proximal end of the first conduit (12) comprises a connector (102) adapted to operatively engage the first conduit with an external fluid supply.

13. The device as recited in any one of claims 1 to 12, wherein the device comprises an intubation device.

14. The device as recited in any one of claims 1 to 13, wherein the device comprises an endobronchial device.
